# EUROPEAN PATENT APPLICATION

(11) **EP 4 494 641 A1**
(43) Date of publication of application: **22.01.2025**
(21) Application number: 23190111.7
(22) Date of filing: 07.08.2023
(51) Int. Cl.: A61K 31/4985, A61P 35/00, A61P 37/00

(54) **SITAGLIPTIN IS A MALT1 INHIBITOR**

(30) Priority: 20.07.2023 GR 20230100598
(71) Applicant: Cloudpharm Private Company, 15125 Marousi (GR); National Hellenic Research Foundation, 11635 Athens (GR)
(72) Inventor: KATSILA MATSOUKA, Theodora, 15233 Chalandri (GR); ZOUMPOULAKIS, Panagiotis, 17676 Kalithea (GR); BAFITI, Paraskevi, 10446 Athens (GR); MATSOUKAS, Minos, 26442 Patras (GR); OUZOUNIS, Sotirios, 12243 Egaleo (GR); PANAGIOTOPOULOS, Vasileios, 12136 Peristeri (GR); GIATRO, Spyridon Marios, 14123 Kiffisia (GR)
(74) Representative: Roukounas, Dimitrios

(57) **Abstract**

Sitagliptin, or a pharmaceutically acceptable salt thereof, for use in the treatment of a disease that can be treated by the inhibition of MALT1.

## Description

### Technical field of the invention

The invention relates to medical uses of the active pharmaceutical ingredient sitagliptin.

### Background of the invention

Sitagliptin (C16H15F6N5O; (3R)-3-amino-1-[3-(trifluoromethyl)-6,8-dihydro-5H-[1,2,4]triazolo[4,3-a]pyrazin-7-yl]-4-(2,4,5-trifluorophenyl)butan-1-one) is the first antidiabetic agent from the class of dipeptidyl peptidase-4 (DPP-4) enzyme inhibitors. It increases the amount of circulating incretins, which stimulate insulin secretion and inhibit glucose production (Choy et al, 2007).

DPP-4 is an enzyme that breaks down incretin hormones such as glucagon-like peptide-1 (GLP-1) and glucose-dependent insulinotropic polypeptide-1 (GIP) (Plosker, 2014). By inhibiting DPP-4, sitagliptin results in improved glucose control. Additionally, sitagliptin decreases the rate at which glucose is produced by the liver. This decreased hepatic glucose production contributes further to improved glycaemic control in patients with type-2 diabetes (Katsuno et al, 2013; Sakura et al, 2016).

Several preclinical studies have also shown that DPP-4 inhibitors can improve endothelial function, reduce oxidative stress, and attenuate inflammation, all of which are key factors in the pathogenesis of cardiovascular diseases (Hanssen and Jandeleit-Dahm, 2019; Liu et al, 2019; Chikata et al, 2022). Clinical trials have also demonstrated that sitagliptin can improve left ventricular function and reduce circulating biomarkers of inflammation and oxidative stress in patients with heart failure and preserved ejection fraction (Zannad et al, 2015). The underlying mechanism for these effects is related to the inhibition of DPP-4, which leads to increased levels of GLP-1 and other vasoactive peptides that enhance endothelial function and reduce inflammation (Kim et al, 2014). Thus, sitagliptin has been shown to have potential benefits for cardiovascular disease patients, including the risk of heart attack and stroke. There is some evidence to suggest that sitagliptin may be of benefit to polycystic ovary syndrome patients in light of insulin resistance and metabolic dysfunction as well as Alzheimer's disease patients regarding cognitive function and disease progression.

Mucosa-associated lymphoid tissue (MALT) lymphoma translocation protein-1, also known as MALT1, is the only human paracaspase (McAllister-Lucas and Lucas, 2008; Israël and Bornancin, 2018). The CARD11-BCL10-MALT1 (CBM) signalosome complex connects proximal antigen receptor signalling to the IkB kinase complex to induce the canonical NF-kB pathway.

The aberrant activation of MALT1 has been associated with NF-kB signalling-dependent neoplasms as MALT1-dependent cleavage of HOIL1 modulates canonical NF-kB signalling (negative feedback termination), while the protease activity of MALT1 itself also controls NF-kB signalling (Quancard et al, 2019; Ruland and Hartjes, 2019). Mutations or aberrant expression of MALT1 have been associated with various lymphoid malignancies, including MALT lymphoma, diffuse large B-cell lymphoma, and mantle cell lymphoma. In recent years, MALT1 has emerged as a potential therapeutic target for the treatment of lymphoid malignancies and several small-molecule inhibitors of MALT1 are currently being developed and tested in preclinical and clinical studies (Vicente-Dueñas et al, 2012; Morishita et al, 2020; Fontán, 2020; Yin et al, 2021; Minderman et al, 2023; Jiang et al, 2023). There is growing evidence to suggest that MALT1 serves as a promising therapeutic target for aggressive brain tumors, such as gliomas. Studies have shown that MALT1 is overexpressed in glioma cells and plays a role in promoting tumor growth and survival. Inhibitors of MALT1 have been shown to induce cell death in glioma cells and inhibit their proliferation in preclinical studies (Jacobs et al, 2020; Liu et al, 2020; Mempel and Krappmann, 2022). MALT1 may also act as a promising therapeutic target for autoimmune diseases, such as rheumatoid arthritis and multiple sclerosis. MALT1 is involved in the activation of T-cells and Inhibitors of MALT1 have shown promise in preclinical studies as potential treatments for these conditions. MALT1 has been also implicated in the development and progression of inflammatory bowel disease, for which MALT1 inhibitors have shown promise in animal models of the disease. MALT1 inhibitors have been studied as potential treatments for chronic obstructive pulmonary disease and asthma taking into account the role of MALT1 in such disease phenotypes.

### Summary of the invention

The present inventors have found that sitagliptin is an inhibitor of MALT1.

Thus, the present invention provides sitagliptin, or a pharmaceutically acceptable salt thereof, for use in the treatment of a disease in a subject, wherein the disease can be treated by the inhibition of MALT1.

The present invention provides also sitagliptin, or a pharmaceutically acceptable salt thereof, for use in the treatment of a disease in a subject, wherein the disease is selected from cancer of the central nervous system (CNS), hematological cancer, aracaspase-dependent immune disease, allergic inflammation, or autoimmune disease.

The present invention provides also a pharmaceutical composition comprising a therapeutically effective amount of sitagliptin, or a pharmaceutically acceptable salt thereof and a pharmaceutically acceptable carrier, for use in the treatment of a disease in a subject, wherein the disease can be treated by the inhibition of MALT1.

The present invention provides also a pharmaceutical composition comprising a therapeutically effective amount of sitagliptin or a pharmaceutically acceptable salt thereof, for use in the treatment of a disease in a subject, wherein the disease is selected from cancer of the central nervous system (CNS), hematological cancer, aracaspase-dependent immune disease, allergic inflammation, or autoimmune disease.

### Brief description of the drawings

Figure 1 shows the anti proliferative effects of MI-2, mepazine and sitagliptin on U87-MG cell growth. Results are mean ± standard deviation of three independent experiments done in triplicates.
Figure 2A shows the inhibition of MALT1 by sitagliptin at 10 µM in patient-derived glioblastoma cells (U3024) that over-express MALT1 resulting in spheroid disintegration (right) when compared to untreated spheroids (left). Figure 2B shows the inhibition of MALT 1 by MI-2, mepazine and sitagliptin in patient-derived glioblastoma cells (U3024).

### Detailed description of the invention

Sitagliptin is an active pharmaceutical known in the prior art for its action as DPP-4 inhibitor.

The present inventors have found that sitagliptin is also a MALT1 inhibitor.

Thus, the present invention provides sitagliptin, or a pharmaceutically acceptable salt thereof, for use in the treatment of a disease in a subject, wherein the disease can be treated by the inhibition of MALT1.

Examples of diseases to be treated according to the present invention include cancer of the central nervous system (CNS), including brain cancer, hematological cancer, aracaspase-dependent immune disease, allergic inflammation and autoimmune disease.

Preferably, the disease to be treated according to the present invention is selected from glioma, medulloblastoma, primary CNS lymphoma, non-Hodgkin's lymphoma, diffuse large B-cell lymphoma, MALT lymphoma, mantle cell lymphoma, allergic inflammation, or multiple sclerosis. More preferably, the disease is selected from glioma, medulloblastoma, primary CNS lymphoma, non-Hodgkin's lymphoma, diffuse large B-cell lymphoma, or MALT lymphoma. Even more preferably, the disease is selected from glioma, medulloblastoma, or primary CNS lymphoma.

The present invention provides also sitagliptin, or a pharmaceutically acceptable salt thereof, for use in the treatment of a disease in a subject selected from CNS cancer, such as brain cancer, hematological cancer, aracaspase-dependent immune disease, allergic inflammation, or autoimmune disease.

Preferably, the disease to be treated by sitagliptin, or a pharmaceutically acceptably salt thereof, is selected from glioma, medulloblastoma, primary CNS lymphoma, non-Hodgkin's lymphoma, diffuse large B-cell lymphoma, MALT lymphoma, mantle cell lymphoma, allergic inflammation, or multiple sclerosis. More preferably, the disease is selected from glioma, medulloblastoma, primary CNS lymphoma, non-Hodgkin's lymphoma, diffuse large B-cell lymphoma, or MALT lymphoma. Even more preferably, the disease is selected from glioma, medulloblastoma, or primary CNS lymphoma.

Preferably, the subject is a mammal, more preferably a human.

Sitagliptin contains an amino group which can form a salt with an acid. Pharmaceutically acceptable salts of sitagliptin are well known in the art. Examples of pharmaceutically acceptable salts of sitagliptin which can be used in the treatment according to the present invention include chloride, bromide, phosphate, nitrate, sulphate, acetate, oxalate, fumarate, citrate and the like.

According to the present invention, sitagliptin, or a pharmaceutically acceptable salt thereof, is typically administered in the form of a pharmaceutical composition. Pharmaceutical compositions comprising sitagliptin, or a pharmaceutically acceptable salt thereof are well known in the art.

For example, the composition may be formulated for oral, parenteral, intramuscular, intravenous, intraperitoneal, subcutaneous, transdermal/intradermal, or inhalation administration. The composition may have different forms, such as solid or liquid forms, for example it can have the form of tablet, capsule, powder, solution, suspension, or emulsion. The composition may comprise one or more excipients, such as solvents, buffering agents, emulsifying agents, preservatives, antioxidants, chelating agents, diluents, binders, disintegrants, lubricating agents and the like, which are well known in the art.

Examples of solvents, include water, saline, phosphate buffered saline, ethanol, isopropyl alcohol and mixtures thereof.

Examples of buffering agents include citric acid monohydrate, sodium citrate, sodium lactate, calcium lactate, acetic acid, sodium acetate, potassium acetate, dibasic potassium phosphate, monobasic potassium phosphate sodium hydrogen phosphate and sodium bicarbonate.

Examples of emulsifying agents include sodium lauryl sulfate, lecithin, polysorbate, sorbitan monooleate, polyethylene glycol, polypropylene glycol, polyvinyl alcohol, poloxamer nonionic surfactants, acacia, agar, alginic acid and sodium alginate.

Examples of preservatives include sodium benzoate, benzyl alcohol, benzalkonium chloride, benzethonium chloride, bronopol, cetrimide, ethanol, and parahydroxy benzoic acids and their alkyl esters.

Examples of antioxidants include sodium metabisulfite, butylated hydroxyanisole, butylated hydroxytoluene and ascorbic acid.

Examples of chelating agents include ethylenediaminetetraacetic acid and salts thereof (e.g., sodium edetate, disodium edetate, trisodium edetate, calcium disodium edetate, and the like), citric acid and salts thereof, fumaric acid and salts thereof, phosphoric acid and salts thereof, and tartaric acid and salts thereof.

Examples of diluents include lactose, sucrose, dextrose, mannitol sorbitol, inositol, calcium carbonate, sodium carbonate, calcium phosphate, dicalcium phosphate, microcrystalline cellulose, kaolin, sodium chloride, dry starch and cornstarch.

Examples of binders include hydroxypropylcellulose, hydroxyethylcellulose, carboxymethylcellulose, dextrose, xylitol, polyvinylpyrrolidone, polyethylene glycol, alginates and gelatin.

Examples of lubricating agents include magnesium stearate, calcium stearate, stearic acid, silica, talc, malt and sodium lauryl sulfate.

According to the present invention, the pharmaceutical composition comprising sitagliptin, or a pharmaceutically acceptable salt thereof may be administered via a variety of routes, including oral, intravenous, intramuscular, subcutaneous, intraventricular, transdermal, intraperitoneal, topical mucosal, nasal, buccal and sublingual route. The route of administration may depend upon various factors including the disease to be treated, the age of the subject and the condition of the subject. The pharmaceutical composition is generally administered daily, or more frequently, such as twice a day, or less frequently, such as every second day, or every week. The frequency of administration and the administered dose depend on various factors, such as the disease, the route of administration, the age of the subject, and the form of the administered composition, and can be determined by a person skilled in the art by using common general knowledge. Preferably, a single dose according to the present invention comprises from 0.01 mg to 1000 mg, more preferably, from 1 mg to 800 mg and even more preferably, from 5 mg to 500 mg sitagliptin, or a pharmaceutically acceptable salt thereof.

### Examples

### Example 1

Sitagliptin exhibits antiproliferative effects on Glioblastoma multiforme (GBM) cell growth

To measure the MALT1-dependent antiproliferative effect of sitagliptin on GMB cell growth and at the same time account for biases, U3024, an established and characterized GBM cell line derived from GBM patient surgical samples was chosen (https://www.hgcc.se/). U3024 is of mesenchymal subtype (female, 73 years old) and is characterized by MALT1 overexpression. Cell lines were maintained in serum-free neural stem cell media to retain tumour-initiating capacity as well as tumour specific phenotypes (Pollard et al, 2009). The U87MG glioma cell line served as a bona fide human glioblastoma cell line. Cell cultures were performed in 3D. As positive controls, two MALT1 inhibitors were included, namely MI-2 and (S)-mepazine. MI-2 (C₁₉H₁₇Cl₃N₄O₃; 2-chloro-N-[4-[5-(3,4-dichlorophenyl)-3-(2-methoxyethoxy)-1,2,4-triazol-1-yl] phenyl] acetamide) is an orthosteric and irreversible MALT1 inhibitor (Fontán et al, 2012). (S)-mepazine (C₁₉H₂₂N₂S; 10-{[(3S)-1-Methyl-3-piperidinyl] methyl}-10H-phenothiazine) is an allosteric MALT1 inhibitor with higher binding affinity and inhibitory activity than (R)-mepazine or (±)-mepazine (Schlauderer et al, 2013). Cell lines were in log phase of growth, harvested and seeded in a 96-well plate at optimum plating density. Next, sitagliptin and positive controls, MI-2 and (S)-mepazine were tested at 0.1 µM, 1 µM, and 10 µM at t=48h. DMSO was <0.05%.

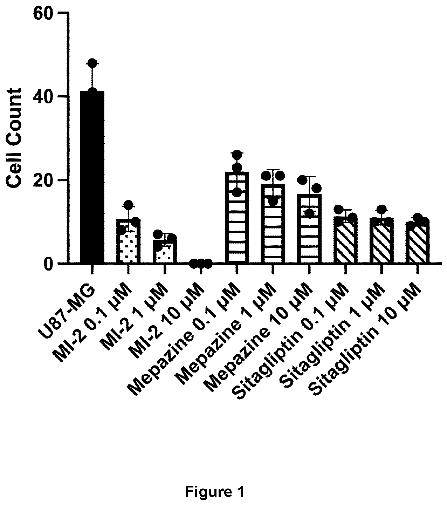

Figure 1 shows the antiproliferative effects of MI-2, mepazine and sitagliptin on U87-MG cell growth. The results are mean ± standard deviation of three independent experiments done in triplicates.

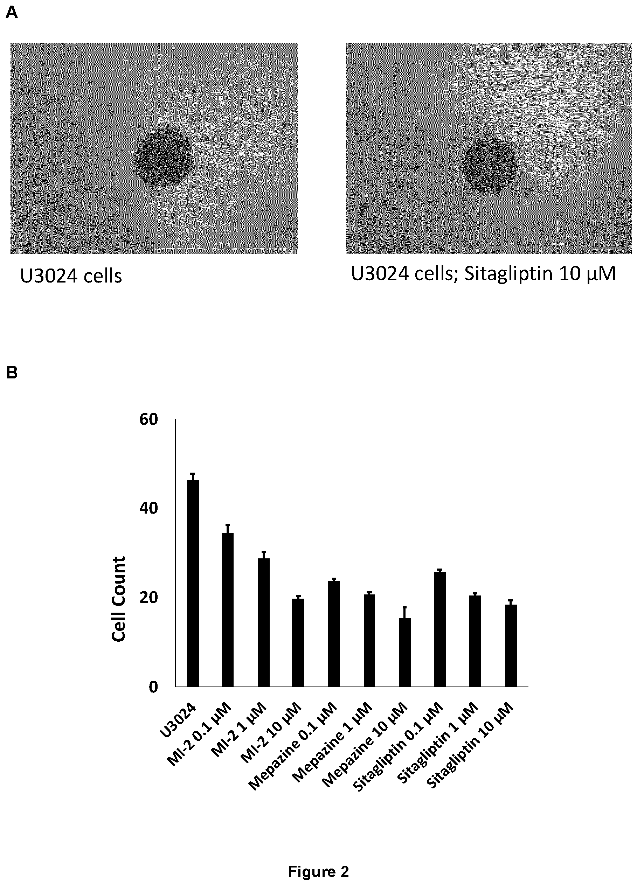

Figure 2A shows the inhibition of MALT1 by sitagliptin at 10 µM in patient-derived glioblastoma cells (U3024) that over-express MALT1 resulting in spheroid disintegration (right) when compared to untreated spheroids (left).

Figure 2B shows the inhibition of MALT 1 by MI-2, mepazine and sitagliptin in patient-derived glioblastoma cells (U3024). Three independent experiments were performed in triplicates.

The above results clearly show that sitagliptin exhibits strong antiproliferative effect on GBM cell growth.

### Example 2

### Sitagliptin inhibits the proteolytic activity of MALT1

Sitagliptin inhibition of MALT1 proteolytic activity was performed at 10 µM. Fluorogenic reactions were monitored in the presence of recombinant full-length human MALT1 and Ac-LRSR-AMC (Ac-Leu-Arg-Ser-Arg-7-amino-4-methylcoumarin) - SEQ ID NO:1 - with excitation/emission wavelengths of 360/460 nm in black, low binding microtiter plates using a microplate reader. Cleavage of the Ac-LRSR-AMC substrate by MALT1 results in the release of AMC and a fluorescent signal. To eliminate false positives due to compound autofluorescence, two time points were measured for each reaction and hence, the fluorescence difference between such time points (T2-T1) was defined as MALT1 activity. The final percent inhibition was calculated with the formula: {[fluorescence_test compound(T2-T1) - fluorescence_negative control (T2-T1)]/ [fluorescence_positive control (T2-T1) - fluorescence_negative control (T2-T1)]} × 100. (S)-mepazine (C₁₉H₂₂N₂S; 10-{[(3S)-1-Methyl-3-piperidinyl] methyl}-10H-phenothiazine) or MI2 (C19H17Cl3N4O3; 2-chloro-N-[4-[5-(3,4-dichlorophenyl)-3-(2-methoxyethoxy)-1,2,4-triazol-1-yl] phenyl] acetamide) were used as positive controls at 10 µM. A so-called buffer only test-condition (i.e. MALT1+Ac-LRSR-AMC substrate+buffer) served as negative control. Sitagliptin was identified as a MALT1 inhibitor using 40% inhibition as a threshold and validated against MI-2 and/or (S)-mepazine. As positive controls, two MALT1 inhibitors were included, namely MI-2 and (S)-mepazine. MI-2 (C₁₉H₁₇Cl₃N₄O₃; 2-chloro-N-[4-[5-(3,4-dichlorophenyl)-3-(2-methoxyethoxy)-1,2,4-triazol-1-yl] phenyl] acetamide) is an orthosteric and irreversible MALT1 inhibitor (Fontán et al, 2012). (S)-mepazine (C₁₉H₂₂N₂S; 10-{[(3S)-1-Methyl-3-piperidinyl] methyl}-10H-phenothiazine) is an allosteric MALT1 inhibitor with higher binding affinity and inhibitory activity than (R)-mepazine or (±)-mepazine (Schlauderer et al, 2013).

EC₅₀ (effective concentration 50%) refers to the concentration of a test-compound at which 50% of its maximum effect is achieved. The term is quite general, regardless of whether a test-compound enhances or diminishes the biological parameter in question. Its equation is a modified version of the IC₅₀ equation, taking the ratio of the response (R) to the maximum response (Rmax): {R/Rmax} = {1/ [1+(E₅₀/I)n]}. In cases where a test-compound completely inhibits a biological activity at high dose, the values of EC₅₀ and IC₅₀ are identical (where Rmax = 100%). However, some test-compounds achieve only partial attenuation of a biological activity, even at high concentrations. In this case, IC₅₀ values may be misleading about potency. Furthermore, efficacious test-compounds have more similar IC₅₀ and EC₅₀ values than their less efficacious counterparts. Of note and in contrast to IC₅₀ values, EC₅₀ values report on the binding affinity of the test-compound (effective concentration 50% is achieved when half of the target protein is bound). Using EC50 values allows the dose-response of test-compounds to be best quantified and reported when complex biological systems are employed, such as cell or animal models.

Cell lines were in log phase of growth, harvested and seeded in a 96-well plate at optimum plating density. Next, cells were incubated with and without test-compounds (0.1 µM - 1 µM - 10 µM) in the presence of positive, negative and untreated controls. EC₅₀ values were determined by Trypan blue dye-exclusion at t=48h and the IC50 calculator (Quest Graph^{™} IC50 Calculator." AAT Bioquest, Inc., 25 Apr. 2023, https://www.aatbio.com/tools/ic50-calculator).

Sitagliptin inhibits the proteolytic activity of MALT1 with an EC₅₀ value of 1.3 µM (Table 1). Results are mean ± standard deviation of three independent experiments done in triplicates.

**Table 1. Sitagliptin, MI-2 and (S)-mepazine EC₅₀ values**

| Test-compounds | EC₅₀ values (µM) |
|---|---|
| Sitagliptin | 1.3 |
| MI-2 | 1.0 |
| (S)-mepazine | 1.0 |

### Example 3

Sitagliptin did not inhibit nor induced CYP1A2, CYP2A6, CYP2B6, CYP2C9, CYP2C19, CYP2D6 and CYP3A4 human CYP450 isoenzymes.

To determine the isozyme-specific CYP450-metabolism, baculosomes^{®} expressing CYP1A2, CYP2A6, CYP2B6, CYP2C9, CYP2C19, CYP2D6 and CYP3A4 human CYP450 isoenzymes were purchased from Thermo Fisher Scientific (Waltham, MA, USA). All reagents were handled and prepared according to the manufacturer's protocol. Sitagliptin was tested at 1 µM. Enzymatic activity in the presence of sitagliptin was assessed based on the kinetic model for CYP1A2, CYP2A6, CYP2B6, CYP2C9, CYP2C19, CYP2D6 and CYP3A4 isoforms. Reactions took place in triplicates at 20_{°}C. Glucose-6-phosphate at 333 mM and glucose-6-phosphate-dehydrogenase at 30 U/mL in 100 mM potassium phosphate, pH 8.0 (regeneration system) converted NADP+ (10 mM in 100 mM potassium phosphate, pH 8.0) into NADPH to initiate the CYP450 reaction. Upon the addition of the fluorescent substrate, immediately (<2 min), the fluorescence signal was monitored over time at suitable excitation and emission wavelengths by Lionheart FX (BioTek, Winooski, VT, USA). CYP450 inhibition (%) was determined based on the reaction rates (fluorescence intensity changes per unit time). A total of n = 60 measurements per minute were acquired for a total time of t = 60 min. % Inhibition= (1 - X/A) × 100%, where: X is the rate observed in the presence of test-compound; A is the rate observed in the presence of negative (solvent, DMSO) control.

Data show that sitagliptin does not inhibit nor induces CYP1A2, CYP2A6, CYP2B6, CYP2C9, CYP2C19, CYP2D6 and CYP3A4 human CYP450 isoenzymes.

### Non-patent literature cited in the description

Avram S, Wilson TB, Curpan R, Halip L, Borota A et al. Nucleic Acids Res 2023, 51(D1): D1276.

Chikata Y, Iwata H, Miyosawa K, Koike T, Yasuda H et al. Sci Rep 2022, 12(1): 5129.

Choy M, Lam S. Cardiol Rev 2007, 15(5): 264.

Fontán L, Yang C, Kabaleeswaran V, Volpon L, Osborne MJ et al. Cancer Cell 2012, 22(6): 812.

Fontán L. Blood Cancer Discov 2020, 1(3_Supplement): IA49.

Hanssen N Mj and Jandeleit-Dahm KA. Diab Vasc Dis Res 2019, 16(4): 303.

Hart! D, de Luca V, Kostikova A, Laramie J, Kennedy S et al. J Transl Med 2021, 19(1): 245.

Israël L and Bornancin F. Cell Mol Immunol 2018, 15(1): 8.

Jiang VC, Liu Y, Lian J, Huang S, Jordan A et al. J Clin Invest 2023, 133(3): e165694.

Jacobs KA, Andre-Gregoire G, Maghe C, Thys A, Li Y et al. EMBO J, 39(1): e102030.

Katsuno T, Ikeda H, Ida K, Miyagawa J-I, Namba M. Endocr J 2013, 60(6): 733.

Kim N-H, Yu T, Lee DH. Biomed Res Int 2014, 2014: 368703.

Liu D, Jin B, Chen W, Yun P. BMC Pharmacol Toxicol 2019, 20(1): 15.

Liu X, Yue C, Shi L, Liu G, Cao Q et al. J Cell Mol Med 2020, 24(13): 7550.

McAllister-Lucas LM and Lucas PC. Nat Immunol 2008, 9(3): 231.

Mempel TR and Krappmann D. J Immunother Cancer 2022, 10(10): e005442.

Minderman M, Lantermans HC, Grüneberg LJ, Cillessen SAGM, Bende RJ et al. Blood Cancer J 2023, 13(1): 37.

Morishita D, Mizutani A, Tozaki H, Arikawa Y, Kameda T et al. Blood 2020, 136 (Supplement 1): 3.

Plosker GL. Drugs 2014, 74(2): 223.

Pollard SM, Yoshikawa K, Clarke ID, Danovi D, Stricker S et al. Cell Stem Cell 2009, 4(6): 568.

Pushpakom S, Iorio F, Eyers PA, Escott KJ, Hopper S. Nat Rev Drug Discov 2019, 18(1): 41.

Quancard J, Klein T, Fung S-Y, Renatus M, Hughes N et al. Nat Chem Biol 2019, 15(3): 304.

Ruland J and Hartjes L. Nat Rev Immunol 2019, 19(2):118.

Sakura H, Hashimoto N, Sasamoto K, Ohashi H, Hasumi S et al. BMC Endocr Disord 2016, 16(1): 70.

Schlauderer F, Lammens K, Nagel D, Vincendeau M, EitelhuberAC et al. Angew Chem Int Ed Engl 2013, 52(39): 10384.

Vicente-Dueñas C, Fontán L, Gonzalez-Herrero I, Romero-Camarero I, Segura Vet al. Proc Natl Acad Sci USA 2012, 109(26): 10534.

Yin W, Nie Z, Dingley K, Trzoss M, Krilov G et al. Blood 2021, 138 (Supplement 1): 1187.

Zannad F, de Ferrari GM, Tuinenburg AE, Wright D, Brugada J et al. Eur Heart J 2015, 36(7): 425.

## Claims

1. Sitagliptin, or a pharmaceutically acceptable salt thereof, for use in the treatment of a disease in a subject, wherein the disease is selected from cancer of the central nervous system (CNS), including brain cancer, hematological cancer, aracaspase-dependent immune disease, allergic inflammation, or autoimmune disease.

2. Sitagliptin, or a pharmaceutically acceptable salt thereof, for use according to claim 1, wherein the disease is selected from glioma, medulloblastoma, primary CNS lymphoma, non-Hodgkin's lymphoma, diffuse large B-cell lymphoma, MALT lymphoma, mantle cell lymphoma, allergic inflammation, or multiple sclerosis.

3. Sitagliptin, or a pharmaceutically acceptable salt thereof, for use according to claim 1 or 2, wherein the disease is selected from glioma, medulloblastoma, primary CNS lymphoma, non-Hodgkin's lymphoma, diffuse large B-cell lymphoma, or MALT lymphoma.

4. Sitagliptin, or a pharmaceutically acceptable salt thereof, for use according to any one of the preceding claims, wherein the disease is selected from glioma, medulloblastoma, or primary CNS lymphoma.

5. Sitagliptin, or a pharmaceutically acceptable salt thereof, for use in the treatment of a disease in a subject, wherein the disease can be treated by the inhibition of MALT1.

6. Sitagliptin, or a pharmaceutically acceptable salt thereof, for use according to claim 5, wherein the disease is selected from CNS cancer, including brain cancer, hematological cancer, aracaspase-dependent immune disease, allergic inflammation, or autoimmune disease.

7. Sitagliptin, or a pharmaceutically acceptable salt thereof, for use according to claim 5 or 6, wherein the disease is selected from glioma, medulloblastoma, primary CNS lymphoma, non-Hodgkin's lymphoma, diffuse large B-cell lymphoma, MALT lymphoma, mantle cell lymphoma, allergic inflammation, or multiple sclerosis.

8. Sitagliptin, or a pharmaceutically acceptable salt thereof, for use according to any one of claims 5 to 7, wherein the disease is selected from glioma, medulloblastoma, primary CNS lymphoma, non-Hodgkin's lymphoma, diffuse large B-cell lymphoma, or MALT lymphoma.

9. Sitagliptin, or a pharmaceutically acceptable salt thereof, for use according to any one of claims 5 to 8, wherein the disease is selected from glioma, medulloblastoma, or primary CNS lymphoma.

10. Sitagliptin, or a pharmaceutically acceptable salt thereof, for use according to any one of the preceding claims, wherein the subject is a mammal, preferably a human.

11. A pharmaceutical composition comprising a therapeutically effective amount of sitagliptin, or a pharmaceutically acceptable salt thereof and a pharmaceutically acceptable carrier for use in the treatment of a disease in a subject, wherein the disease can be treated by the inhibition of MALT1.

12. The pharmaceutical composition for use according to claim 11, wherein the disease is selected from CNS cancer, hematological cancer, aracaspase-dependent immune disease, allergic inflammation, or autoimmune disease.

13. The pharmaceutical composition for use according to claim 11 or 12, wherein the composition is suitable for oral, parenteral, intramuscular, intravenous, intraperitoneal, subcutaneous, transdermal or inhalation administration.

14. The pharmaceutical composition for use according to any one of claims 11 to 13, wherein the administered dose of sitagliptin, or a pharmaceutically acceptable salt thereof, is from 0.01 mg to 1000 mg.

15. The pharmaceutical composition for use according to any one of claims 11 to 14, wherein the administered dose of sitagliptin, or a pharmaceutically acceptable salt thereof, is from 5 mg to 500 mg.
